# EUROPEAN PATENT SPECIFICATION

(11) **EP 2 101 734 B1**
(45) Date of publication and mention of the grant of the patent: **23.01.2013**
(21) Application number: 07854893.0
(22) Date of filing: 30.11.2007
(51) Int. Cl.: A61K 9/133

(54) **Vesicles of self-assembling block copolymers and methods for making and using the same**
Vesikel von selbst zusammensetzenden Blockcopolymeren und Verfahren zu ihrer Herstellung und Verwendung
Vésicules de copolymères séquencés à auto-assemblage, et procédés de fabrication et d'utilisation de ceux-ci

(30) Priority: 01.12.2006 US 872078 P
(43) Date of publication of application: 23.09.2009
(73) Proprietor: The Regents of the University of California, Oakland, CA 94607-5200 (US)
(72) Inventor: DEMING, Timothy J., Los Angeles, CA 90049 (US); KAMEI, Daniel T., Monterey Park, CA 91755 (US); HOLOWKA, Eric P., Santa Monica, CA 90405 (US); SUN, Victor Z., Los Angeles, CA 90034 (US)
(74) Representative: Jones, Nicholas Andrew
(86) International application number: PCT/US2007/086161
(87) International publication number: WO 2008/070571

(56) References cited:
- WO-A1-2006/113667
- WO-A2-01/94379
- US-A1- 2003 147 958
- HOLOWKA ET AL: "Charged Polypeptide vesicles with controllable diameter", JOURNAL OF THE AMERICAN CHEMICAL SOCIETY, AMERICAN CHEMICAL SOCIETY, WASHINGTON, DC; US, vol. 127, 1 January 2005 (2005-01-01), pages 12423-12428, XP008110149, ISSN: 0002-7863, DOI: 10.1021/JA053557T
- HOLOWKA ET AL. J. AM. CHEM. SOC. vol. 127, 2005, pages 12423 - 12428, XP008110149

## Description

### INTRODUCTION

Polymeric vesicles are a relatively new class of nanoscale self-assembled materials that show great promise as robust encapsulants. Compared to liposomes, use of polymeric building blocks for membrane formation allows increased stability, stimuli responsiveness and chemical diversity, which may prove advantageous for drug delivery applications (Discher, D. E., Eisenberg, A. Polymer Vesicles, Science 297, 967-973 (2002)). For example, polypeptide vesicles composed of either lysine-leucine (poly(L-lysine)₆₀-*block*-poly(L-leucine)₂₀, K₆₀L₂₀) or glutamate-leucine (poly(L-glutamic acid)₆₀-*block*-poly(L-leucine)₂₀, E₆₀L₂₀) diblock copolypeptide amphiphiles have been reported. (Holowka, E. P., Pochan, D. J., Deming, T. J. Charged Polypeptide Vesicles with Controllable Diameter, J. Amer. Chem. Soc. 127, 12423-12428 (2005)). These vesicular assemblies formed in aqueous solution due to a combination of the α-helical hydrophobic segments that favor formation of flat membranes, and the highly charged hydrophilic segments that impart solubility and fluidity to these membranes. The resulting materials show great promise as biomimetic encapsulants that can be prepared with diameters ranging from 50 to 1000 nm, are stable up to 80 °C, can retain polar contents without leakage, and are readily and reproducibly prepared in large quantities (Holowka et al., supra).

In recent years, many groups have utilized protein transduction domains (PTD) to enhance intracellular delivery of cargos (Rothbard, J.B., Jessop, T.C., Wender, P.A. Adaptive translocation: the role of hydrogen bonding and membrane potential in the uptake of guanidinium-rich transporters into cells, Adv. Drug Deliv. Rev. 57, 495-504 (2005); Futaki, S. Membrane-permeable arginine-rich peptides and the translocation mechanisms, Adv. Drug Deliv. Rev. 57, 547-558 (2005); Brooks, H., Lebleu, B., Vivès, E. Tat peptide-mediated cellular delivery: back to basics, Adv. Drug Deliv. Rev. 57, 559-577 (2005); and Wadia, J.S., Dowdy, S.F. Transmembrane delivery of protein and peptide drugs by TAT-mediated transduction in the treatment of cancer, Adv. Drug Deliv. Rev. 57, 579-596 (2005)), a well studied example being the arginine-rich segment (residues 49-57: RKKRRQRRR) of the transactivator of transcription for HIV-1, HIV-1 Tat (Brooks et al., supra). In related studies, it was found that the Tat sequence could be replaced with a simple nonamer of arginine (Calnan, B.J., Tidor, B., Biancalana, S., Hudson, D., Frankel, A.D. Arginine-mediated RNA recognition: the arginine fork, Science 252, 1167-1171 (1991)), showing that the guanidinium residues of arginine are the essential component of this sequence's ability to transport cargos into cells (Mitchell, D.J., Kim, D.T., Steinman, L., Fathman, C.G., Rothbard, J.B. Polyarginine enters cells more efficiently than other polycationic homopolymers, J. Peptide Res. 56, 318-325 (2000); Rothbard, J.B., Garlington, S., Lin, Q., Kirshberg, T., Kreider, E., McGrane, L., Wender, P.A., Khavari, P. A. Conjugation of arginine oligomers to cyclosporin A facilitates topical delivery and inhibition of inflammation, Nature Medicine 6, 1253-1257 (2000)). Since this discovery, many groups have prepared chemical conjugates of guanidinium rich PTDs with drugs, oligonucleotides, proteins, nanoparticles, and liposomes, and successfully delivered them into a broad variety of cell types both *in vitro* and *in vivo* (Rothbard et al., supra; Futaki et al., supra; Brooks et al., supra and Wadia et al., supra).

The use of liposomes functionalized with guanidinium groups for intracellular delivery of therapeutics holds many advantages over chemical conjugation of the therapeutic directly to the PTD (Torchilin, V.P., Rammohan, R., Weissig, V., Levchenko, T.S. TAT peptide on the surface of liposomes affords their efficient intracellular delivery even at low temperature and in the presence of metabolic inhibitors, Proc. Natl. Acad. Sci. USA 98, 9786-8791 (2001); Tseng, Y-L., Liu, J-J., Hong, R-L. Translocation of liposomes into cancer cells by cell-penetrating peptides Penetratin and Tat: a kinetic and efficacy study, Mol. Pharmacol. 62, 864-872 (2002)). Aside from not having to create a degradable chemical linkage to the therapeutic, vesicles are able to carry much larger cargos and even complex mixtures of therapeutics inside the aqueous lumen. The major drawback of lipid based vesicles is their poor stability, which may be compromised even further by attachment of the PTD sequences. The PTD-functionalized lipid vesicles may lose their contents upon storage, or upon binding of the PTD to the cell surface. Polymeric vesicles are known to be very robust and able to encapsulate both hydrophilic and hydrophobic species (Discher et al., supra; Bermudez, H., Brannan, A.K., Hammer, D.A., Bates, F.S., Discher, D.E. Molecular weight dependence of polymersome membrane structure, elasticity, and stability, Macromolecules 35, 8203-8208 (2002)), but most also suffer from their inert polymer building blocks, which require subsequent chemical functionalization with PTDs.

### SUMMARY

Vesicles of self-assembling block copolymers, e.g., diblock copolypeptides, as well as methods of making and using the same, are provided. Vesicles of the invention have a shell made up of block copolymers that include an intracellular transduction hydrophilic domain and a hydrophobic domain. Self-assembling block copolymers are also provided that comprise an intracellular transduction hydrophilic domain and a hydrophobic domain. In certain embodiments, the vesicles include an encapsulated active agent, e.g., a diagnostic or therapeutic agent. The vesicles find use in a variety of different applications, including the intracellular delivery of active agents, e.g., therapeutic and diagnostic agents.

### BRIEF DESCRIPTION OF THE FIGURES

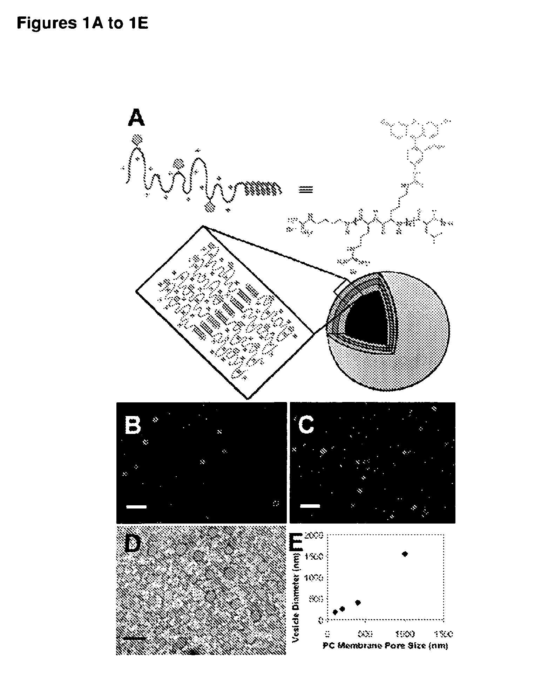

**Figures 1** **A to 1 E.** Formation and properties of R₆₀L₂₀ vesicles. (A) Schematic of proposed self-assembly of R₆₀L₂₀ vesicles. (B) LSCM image of 1.0 µm extruded vesicles (Bar = 5 µm). (C) LSCM image of vesicles containing Texas Red labeled dextran (total solution concentration = 1 µM) (Bar = 5 µm). (D) TEM image of negatively stained vesicles that had been extruded through a 100 nm Nucleopore polycarbonate (PC) membrane filter (Bar = 200 nm). (E) Vesicle diameters determined using DLS after extrusion through different PC membrane filters (0.1, 0.2, 0.4, and 1.0 µm).

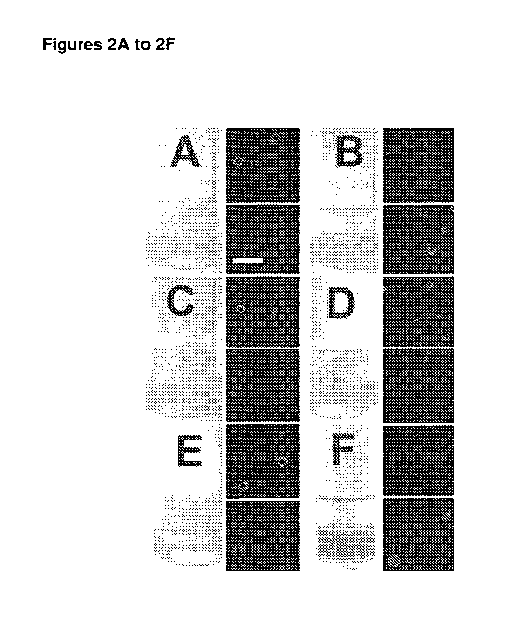

**Figures 2A to 2F****.** Transport of polypeptide vesicles across bulk membranes. Visual and LSCM images of (A) 1 % (w/v) R₆₀L₂₀ vesicle suspension in a 1:1 aqueous buffer (0.5 mL; 10 mM NaH₂PO₄, 100 mM NaCl, pH 7.4)/chloroform mixture, (B) 1 % (w/v) R₆₀L₂₀ vesicle suspension in aqueous buffer/chloroform mixture + EYPG (10 mM), (C) Chloroform layer from sample in (B) added to aqueous sodium sulfate solution (10 mM), (D) 1 % (w/v) R₆₀L₂₀ vesicle suspension in aqueous buffer/chloroform mixture + EYPC (10 mM), (E) 1 % (w/v) K₆₀L₂₀ vesicle suspension in aqueous buffer/chloroform mixture + EYPG (10 mM). Scale Bar for LSCM images = 5 µm, (F) 1 % (w/v) R₆₀L₂₀ vesicles containing Texas Red labeled dextran (total solution concentration = 1 µM) suspended in aqueous buffer/chloroform mixture + EYPG (10 mM).

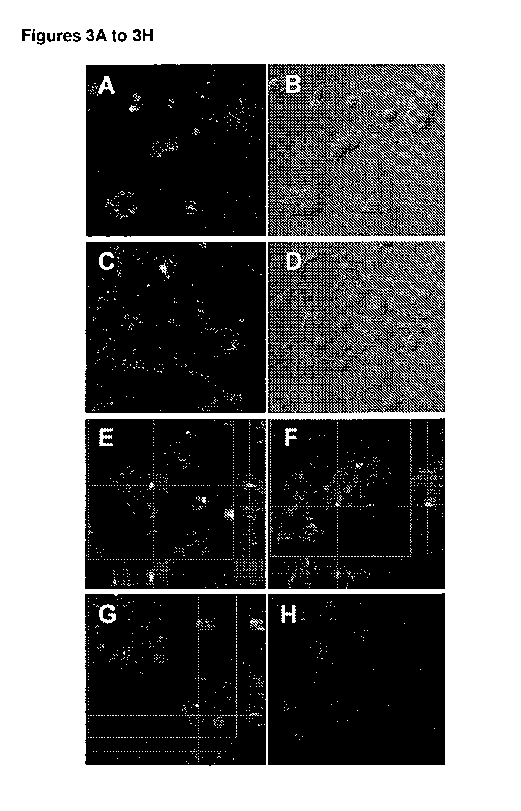

**Figures 3A to 3H** **.** Transport of polypeptide vesicles into cells *in vitro.* (A) LSCM and (B) DIC images of T84 cells after 2.5 hr incubation with R₆₀L₂₀ vesicles (green; 100 µM) containing Texas Red labeled dextran (red; total solution concentration = 1 µM) at 37 ºC without serum. (C) LSCM and (D) DIC images of HULEC-5A cells after 2.5 hr incubation with R₆₀L₂₀ vesicles (green) containing Texas Red labeled dextran (red) at 37 ºC without serum. Three dimensional LSCM reconstructions of T84 cells after incubation with R₆₀L₂₀ vesicles (green) containing Texas Red labeled dextran (red) for 5 hr (E) at 37 ºC without serum, (F) at 37 ºC with serum, and (G) at 0 ºC without serum. (H) LSCM image of T84 cells after incubation with FITC-labeled K₆₀L₂₀ vesicles (100̃ µM) for 5 hr at 37 ºC without serum.

### DETAILED DESCRIPTION

Vesicles of self-assembling block copolymers, e.g., diblock copolypeptides, as well as methods of making and using the same, are provided. Vesicles of the invention have a shell made up of block copolymers that include an intracellular transduction hydrophilic domain and a hydrophobic domain. Also provided are self-assembling block copolymers that comprise an intracellular transduction hydrophilic domain and a hydrophobic domain. In certain embodiments, the vesicles include an encapsulated active agent, e.g., a diagnostic or therapeutic agent. The vesicles find use in a variety of different applications, including the intracellular delivery of active agents, e.g., therapeutic and diagnostic agents.

Before the present invention is described in greater detail, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges and are also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, representative illustrative methods and materials are now described.

The citation of any publication is for its disclosure prior to the filing date and should not be construed as an admission that the present invention is not entitled to antedate such publication by virtue of prior invention. Further, the dates of publication provided may be different from the actual publication dates which may need to be independently confirmed.

It is noted that, as used herein and in the appended claims, the singular forms "a", "an", and "the" include plural referents unless the context clearly dictates otherwise. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

As will be apparent to those of skill in the art upon reading this disclosure, each of the individual embodiments described and illustrated herein has discrete components and features which may be readily separated from or combined with the features of any of the other several embodiments without departing from the scope or spirit of the present invention. Any recited method can be carried out in the order of events recited or in any other order which is logically possible.

In further describing the invention, embodiments of the vesicles of the invention will be reviewed first in greater detail, followed by a discussion of embodiments of compositions that include the vesicles, as well as a review of aspects of making and using the vesicle compositions.

### VESICLES

Aspects of the invention include vesicles that are made up of a shell encapsulating a polar fluid medium. The shell may have a variety of different configurations, but in certain embodiments is spherical. In certain embodiments, the polar fluid medium is an aqueous medium, e.g., water. The vesicles of certain embodiments of the invention are nano-dimensioned vesicles, where the vesicles have, in certain embodiments, a diameter ranging from about 50 to about 1000 nm, such as from about 75 to about 500 nm. The vesicles of the invention may be stable under a variety of conditions, including at temperatures up to about 80ºC or higher. By "stable" is meant that the vesicles do not lose their integrity and do not leak, at least not to any substantial extent, their contents, even for extended periods of time, such as 1 week or longer, 1 month or longer, 2 months or longer, 6 months or longer (when maintained under the conditions analogous to those reported in the experimental section below). In addition, the vesicles are non-toxic, by which is meant that the vesicles exhibit little or no toxicity as determined using the toxicity assay described in the Experimental section, below.

The shell component of vesicles according to embodiments of the invention is one that is made up of self-assembling block copolymers, where the block copolymers (such as copolypeptides described below) may be viewed as amphiphilic. The shell may be made up of a single type of self-assembling block copolymer, such that it is homogenous with respect to the self-assembling block copolymer. Alternatively, the shell may be made up of two or more different types of self-assembling block copolymers. e.g., three or more, four or more, five or more, etc., different types of block copolymers, such that the shell is heterogeneous with respect to the block copolymer. Any two given block copolymers are considered different from each other if their residue sequence differs by at least one residue.

The copolymers making the up the shell of the vesicles are self-assembling block copolymers. By "self-assembling" is meant that the copolymers can, under appropriate conditions, interact with each other to produce the subject vesicle structures, e.g., spherical structures, such as the structure shown in Figure 1.

Aspects of the self-assembling block copolymers include a first hydrophobic domain and a second hydrophilic intracellular transduction domain. By "intracellular transduction domain" is meant a region of the copolymer which serves to enhance or facilitate entry of the vesicle into the interior of a cell. A domain is considered to be an intracellular transduction domain if it enhances entry of the vesicle into the interior of a cell by about 2-fold or more, such as by about 5-fold or more, including by about 10-fold or more, as compared to a suitable control, e.g., as determined using the assays described in the Experimental section below. For instance, it is to be understood that such self-assembling block copolymers exclude the self-assembling poly-L-lysine-*block*-poly-L-leucine copolymers, and poly-L-glutamate-*block*-poly-L-leucine copolymers, as such copolymers do not include such an intracellular transduction domain.

In certain embodiments, the self-assembling block copolymers alone or when comprised a s a vesicle include a first hydrophobic domain and a second hydrophilic intracellular transduction domain, wherein the self-assembling block copolymers are minimally cytoloxic. In a related embodiment, the second hydrophilic intracellular transduction domain by itself is minimally cytotoxic. By "minimally cytotoxic" is intended maintenance of cell viability as compared to a suitable control, e.g., as determined using the assays described in the Experimental section below.

Of interest in certain embodiments are self-assembling block copolymers that include a first hydrophobic domain and a second hydrophilic intracellular transduction domain, wherein the second hydrophilic intracellular transduction domain is polycationic. In a related embodiment, the second hydrophilic intracellular transduction domain is polycationic and is by itself minimally cytotoxic. Of specific interest are self-assembling block copolymers or vesicles thereof that include a first hydrophobic domain and a second hydrophilic intracellular transduction domain, wherein the second hydrophilic intracellular transduction domain is polycationic, and wherein the self-assembling block copolymers are minimally cytotoxic.

The lengths of the first and second domains may be the same or different. In certain embodiments, the length of the second domain is different from the length of the first domain, e.g., where the second domain has a length that is about 2 to about 8, such as about 2 to about 4, times longer than the length of said first domain.

In addition to having a first and second domain, the copolymers may or may not include one or more additional domains, e.g., 2 or more additional domains. If present, such domains may be positioned between the first and second domains, such that the first and second domains make up the first and second terminus of the copolymer.

In certain embodiments, the different domains are polypeptide domains, such that the block copolymer is a block copolypeptide. In these embodiments, the first hydrophobic domain is a homopolypeptidic domain, by which is meant that the domain is made up of identical amino acid residues, or a heteropolypeptidic domain, by which is meant that the domain is made up of two or more different amino acid residues. The length of the first polypeptidic hydrophobic domain may vary, and in certain embodiments ranges from about 5 to about 50 residues, such as from about 10 to about 30 residues, including from about 15 to about 25 residues, e.g., 20 residues. In certain embodiments, this domain is not a racemic domain.

For the first hydrophobic domain, non-polar amino acid residues, e.g., phenylalanine, leucine, valine, isoleucine, alanine, methionine, are employee, with any given domain in certain embodiments containing from 1 to 3 or more of these residues in a statistically random sequence. In certain embodiments, the first hydrophobic domain is a poly-leucine (polyL) domain. In certain embodiments, the polyL domain is 20 residues long, such that it is L₂₀.

As with the first domain, the second hydrophilic domain may be a homopolypeptidic domain, by which is meant that the domain is made up of identical amino acid residues, or a heteropolypeptidic domain, by which is meant that the domain is made up of two or more different amino acid residues. The length of the second polypeptidic hydrophilic domain may vary, and in certain embodiments ranges from about 30 to about 120 residues, such as from about 40 to about 80 residues, including from about 50 to about 80 residues, e.g., 60 residues. In certain embodiments, this domain is not a racemic domain.

For the second hydrophilic domain, polar amino acid residues that can impart intracellular transduction properties to the vesicle, e.g., glutamic acid, aspartic acid, arginine, histidine, lysine, ornithine, are employed, with any given domain in certain embodiments containing from 1 to 3 or more of these residues in a statistically random or block sequence. In certain embodiments, the second hydrophilic domain is a poly-arginine (polyR) domain. In certain embodiments, the polyR domain is 60 residues long, such that it is R₆₀.

In certain embodiments, the shell component of the vesicles of the invention is made up of a single type of self-assembling diblock copolypeptide, where the second domain has a length (in terms of amino acid residues) that is about 2 to 4 times longer than the length of said first domain. In certain of these embodiments, the diblock copolypeptide is a R₆₀L₂₀.

As reviewed above, the vesicles include within the shell component a polar fluid medium, such as an aqueous medium. In certain embodiments, the aqueous medium present in the shell includes an active agent, such that the vesicle includes an amount of an encapsulated active agent. The active agent may vary greatly, where in certain embodiments the active agent is a diagnostic agent, e.g., contrast agent, fluorescent protein, etc., and in other embodiments the active agent is a therapeutic agent, e.g., a drug.

For example, the vesicles of the present invention may be used for medical applications, wherein the cargo to be delivered can be drug molecule(s), therapeutic compound(s), radioactive compound(s), chemotherapy agent(s), DNA/RNA, proteins, or MRI contrast agents. The mode of delivery can include aerosol delivery to lungs via inhalation, subcutaneous injection, ingestion, transdermal delivery (as ointment), e.g., as reviewed in greater detail below. The vesicles also may be used for other applications wherein the cargo to be delivered can be a reagent, such as a research reagent (e.g., serum proteins, growth factors, inhibitors, radioactive compound(s), DNA/RNA, proteins, steroids, sterols, diagnostic agents etc.) or an industrial reagent (e.g., anti-microbials, anti-fungals, pesticides, herbicides, fertilizers etc.). The mode of delivery of such reagent can include any form suitable for contacting a cell of interest, e.g., liquid, powder, emulsion, cream, spray and the like.

Thus a variety of agents can be incorporated covalently or non-covalently into or in association with the subject vesicles with high loads. The resulting vesicles can be used for a wide variety of in vitro and in vivo applications (e.g., delivery of a cargo / payload of an agent of interest into a cell in vitro or in vivo). For instance, in certain embodiments, an agent of interest may be loaded in a vesicle by a non-covalent manner such that the agent is dispersed within the polar medium or associated with the vesicle through a non-covalent relationship with an internal or external surface of the vesicle, embedded in the vesicle wall, or combination thereof. In other embodiments, one or more of the self-assembling block copolymers of a vesicle may be covalently modified with an agent of interest. When covalently attached, the agent may be attached to a residue of the vesicle through a biodegradable bond, such as a disulfide or ester, which bond may induce a linker or spacer on either or both sides. In some embodiments, the vesicles may include both covalent and non-covalently attached agent of interest, as well as single and multiple different payloads, depending on a give end use. In yet other embodiments, the vesicles may be modified with a targeting ligand that routes the vesicle to a specific location for delivery of its cargo (e.g., the hydrophilic intracellular transduction domain can be attached to a targeting ligand that directs the vesicle to a particular receptor, cell, extracellular matrix component, tissue, organ and the like).

As noted above, the vesicles of the invention may be exploited as medical, research and industrial tools for intracellular delivery of a cargo of interest to cells and cell lines. In addition to their use in therapeutic and diagnostic medicine, for instance, the vesicles are well suited as tools for delivering a reagent(s) for modulating cell growth, apoptosis, differentiation, stasis etc. (e.g., intracellular delivery of serum proteins, growth factors, inhibitors, therapeutics etc.), for facilitating cell-based assays (e.g., intracellular delivery of ion indicators, reactive dyes and chemicals, imaging and contrast agents, primary or secondary detection and/or quantitation components), and a wide range of other cell-based applications in genomics, proteomics and microbiology, immunology, biochemistry, and molecular and cell biology in general (e.g., flow cytometry, transfection, staining, cell culturing and the like).

Unlike other intracellular transduction systems (e.g., TAT-drug conjugates), the block copolymers of the present invention spontaneously self-assemble into vesicles when exposed to a poplar medium, such as an aqueous solution. The vesicles are highly stable, can be adjusted to possess various cargo volumes and internal/external surface properties, and also form strong but reversible complexes with non-covalently attached hydrophilic molecules. A significant advantage of such vesicles is that the hydrophilic intracellular transduction domain facilitates both interaction with hydrophilic payloads as well as transport of the vesicles across cell membranes for uptake and intracellular delivery of the vesicles' cargo. The vesicles in and of themselves are also minimally cytotoxic.

Another advantage is that the vesicles can be adapted to carry hydrophobic payloads (e.g., steroids, sterols, dyes such as 5-dodecanoylaminofluorescein, drugs such as paclitaxel etc.), for example, by covalent attachment or admixing a hydrophobic cargo of interest with a suitable amphiphilic surfactant for its dispersion or containment in a polar medium suitable for encapsulation into a vesicle of the invention. Examples of amphiphilic surfactants for this purpose include, for instance, polyethoxylated fatty acids, such as the PEG-fatty acid monoesters and diesters of lauric acid, oleic acid, and stearic acid (as well as PEG-glycerol fatty acid esters of lauric acid, oleic acid, and stearic acid), amphiphilic transesterification products of oils and alcohols, sterols and sterol derivatives, oil-soluble vitamins, such as vitamins A, D, E, K, etc., polyglycerol esters of fatty acids, as well as mixtures of surfactants such as propylene glycol fatty acid esters and glycerol fatty acid esters, amphiphilic esters of sugars such as sucrose monopalmitate and sucrose monolaurate, sucrose monostearate, sucrose distearate, amphiphilic esters of lower alcohols (C2 to C4) and fatty acids (C8 to C8) and the like.

An aspect of the vesicles of the invention is their capacity to incorporate water-soluble cargos and deliver them across a cell membrane into the intracellular environment with high fidelity. Of particular interest are vesicles loaded with a water-soluble active agent. The term "water-soluble active agent" refers to compounds that are soluble in water or have an affinity for water or an aqueous solution, and generally exhibit a given activity by itself but may be in a masked form, such as a prodrug. Such agents may include biologically active compounds such as peptides, proteins, nucleic acids, therapeutic agents, diagnostic agents, and non-biological materials such as pesticides, herbicides, and fertilizers.

Illustrative examples of water-soluble active agent compounds that can be used in the vesicle systems of the present invention are represented by various categories of agents that include, but are not limited to: imaging or diagnostic agents, analgesics, anti-inflammatory agents, antihelminthics, anti-arrhythmic agents, anti-bacterial agents, anti-viral agents, anti-coagulants, anti-depressants, anti-diabetics, anti-epileptics, anti-fungal agent, anti-gout agents, anti-hypertensive agents, anti-malarials, anti-migraine agents, anti-muscarinic agents, anti-neoplastic agents, erectile dysfunction improvement agents, immunosuppresants, anti-protozoal agents, anti-thyroid agents, anxiolytic agents, sedatives, hypnotics, neuroleptics, .beta.-blockers, cardiac inotropic agents, corticosteroids, diuretics, anti-parkinsonian agents, gastro-intestinal agents, histamine receptor antagonists, keratolytics, lipid regulating agents, anti-anginal agents, Cox-2 inhibitors, leukotriene inhibitors, macrolides, muscle relaxants, anti-osteoporosis agents, anti-obesity agents, cognition enhancers, anti-urinary incontinance agents, nutritional oils, anti-benign prostate hypertrophy agents, essential fatty acids, non-essential fatty acids, and mixtures thereof. Likewise, the water-soluble active agent can be a cytokine, a peptidomimetic, a peptide, a protein, a toxoid, a serum, an antibody, a vaccine, a nucleoside, a nucleotide, a portion of genetic material, a nucleic acid, or a mixture thereof. Suitable water-soluble active agents may also include hydrophilic polymers like starch, dextran, polyvinyl alcohol, polyvinyl-pyrrolidone, dextrin, xanthan or partly hydrolyzed cellulose oligomers and the like.

Specific, non-limiting examples of suitable water-soluble active agents as therapeutics or prophylactics include: acarbose; acyclovir; acetyl cysteine; acetylcholine chloride; alatrofloxacin; alendronate; alglucerase; amantadine hydrochloride; ambenomium; amifostine; amiloride hydrochloride; aminocaproic acid; amphotericin B; antihemophilic factor (human); antihemophilic factor (porcine); antihemophilic factor (recombinant); aprotinin; asparaginase; atenolol; atracurium besylate; atropine; azithromycin; aztreonam; BCG vaccine; bacitracin; becalermin; belladona; bapridil hydrochloride; bleomycin sulfate; calcitonin human; calcitonin salmon; carboplatin; capecitabine; capreomycin sulfate; cefamandole nafate; cefazolin sodium; cefepime hydrochloride; cefixime; cefonicid sodium; cefoperazone; cefotetan disodium; cefotaxime; cefoxitin sodium; ceftizoxime; ceftriaxone; cefuroxime axetil; cephalexin; cephapirin sodium; cholera vaccine; chorionic gonadotropin; cidofovir; cisplatin; cladribine; clidinium bromide; clindamycin and clindamycin derivatives: ciprofloxacin; clodronate; colistimethate sodium; colistin sulfate; corticotropin; cosyntropin; cromolyn sodium; cytarabine; dalteparin sodium; danaparoid; desferrioxamine; denileukin diftitox; desmopressin; diatrizoate meglumine and diatrizoate sodium; dicyclomine; didanosine; dirithromycin; dopamine hydrochloride; dornase alpha; doxacurium chloride; doxorubicin; etidronate disodium; enalaprilat; enkephalin; enoxaparin; enoxaparin sodium; ephedrine; epinephrine; epoetin alpha; erythromycin; esmolol hydrochloride; factor IX; famciclovir; fludarabine; fluoxetine; foscamet sodium; ganciclovir; granulocyte colony stimulating factor; granulocyte-macrophage stimulating factor; recombinant human growth hormones; bovine growth hormone; gentamycin; glucagon; glycopyrolate; gonadotrophin releasing hormone and synthetic analogs thereof; GnRH; gonadorelin; grepafloxacin; haemophilus B conjugate vaccine; Hepatitis A virus vaccine inactivated; Hepatitis B virus vaccine inactivated; heparin sodium; indinavir sulfate; influenza virus vaccine; interleukin-2; interleukin-3; insulin-human; insulin lispro; insulin procine; insulin NPH; insulin aspart; insulin glargine; insulin detemir; interferon alpha; interferon beta; ipratropium bromide; ifosfamide; Japanese encephalitis virus vaccine; lamivudine; leucovorin calcium; leuprolide acetate; levofloxacin; lincomycin and lincomycin derivatives; lobucavir; lomefloxacin; loracarbef; mannitol; measles virus vaccine; meningococcal vaccine; menotropins; mepenzolate bromide; mesalamine; methenamine; methotrexate; methscopolamine; metformin hydrochloride; metoprolol; mezocillin sodium; mivacurium chloride; mumps viral vaccine; nedocromil sodium; neostigmine bromide; neostigmine methyl sulfate; neurontin; norfloxacin; octreotide acetate; ofloxacin; olpadronate; oxytocin; pamidronale disodium; pancuronium bromide; paroxetine; perfloxacin; pentamidine isethionate; pentostatin; pentoxifylline; periciclovir; pentagastrin; phentolamine mesylate; phenylalanine; physostigmine salicylate; plague vaccine; piperacillin sodium; platelet derived growth factor; pneumococcal vaccine polyvalent; poliovirus vaccine (inactivated); poliovirus vaccine live (OPV); polymyxin B sulfate; pralidoxime chloride; pramlintide; pregabalin; propafenone; propantheline bromide; pyridostigmine bromide; rabies vaccine; residronate; ribavarin; rimantadine hydrochloride; rotavirus vaccine; salmeterol xinafoate; sincalide; small pox vaccine; solatol; somatostatin; sparfloxacin; spectinomycin; stavudine; streptokinase; streptozocin; suxamethonium chloride; tacrine hydrochloride; terbutaline sulfate; thiopeta; ticarcillin; tiludronate; timolol; tissue type plasminogen activator; TNFR:Fc; TNK-tPA; trandolapril; trimetrexate gluconate; trospectinomycin; trovafloxacin; tubocurarine chloride; tumor necrosis factor; typhoid vaccine live; urea; urokinase; vancomycin; valacyclovir; valsartan; varicella virus vaccine live; vasopressin and vasopressin derivatives; vecuronium bromide; vinblastine; vincristine; vinorelbine; vitamin B12; warfarin sodium; yellow fever vaccine; zalcitabine; zanamivir; zolendronate; zidovudine; pharmaceutically acceptable salts, isomers and derivatives thereof; and mixtures thereof.

A variety of diagnostic agents also can be incorporated covalently or non-covalently into the subject vesicles with high loads. Diagnostic agents of particular interest include, but are not limited to, a detectable label or a reporter ligand, which includes both active and passive reporter ligands such as a component of a fluorescence resonance energy transfer (FRET) detection system, spin-trap agents, quantum dots, chelated agents, contrast agents, dyes, radiolabels, peptides, nucleic acids, antibodies, antibody fragments and the like. Vesicles loaded with diagnostic agents can be used in connection with a variety of detection and imaging modalities, such as those involving standard analytic and/or separation-based detection modalities (e.g., chromatography, Enzyme-Linked ImmunoSorbent Assays (ELISA) etc.), as well as those based on less invasive modalities such as gamma-scintigraphy, magnetic resonance imaging and computed tomography.

For instance, the vesicles can be loaded with chelated or bifunctional chelated agents (e.g., covalent linkage group coupled to a targeting moiety such as an antibody, antibody fragment, peptide or hormone and a chelating group for the metal) and used (depending on the particular agent selected and modality of administration) for angiography (radiographic study of the vascular system), urography (radiographic study of the urinary tract), pyelogram (pelvis and the kidney and ureters), cystogram (urinary bladder), bronchography (radiographic study of the lungs and bronchi), upper GI series or "barium shallow" (radiographic study of the pharynx, esophagus, stomach, duodenum, small intestine), lower GI series or barium enema (radiographic study of the large bowel (colon) and rectum), cholecystography (radiographic study following introduction of contrast agents either orally or IV of the structure of the gall bladder and bile ducts), myelography (radiological study of the spinal cord), salpingography (radiological study of the fallopian tubes), hysterosalpingography (radiographic study of the uterus and fallopian tubes), sialography (radiological study of the salivary glands and ducts), arthrography (radiological study of the joints), discography (radiological study of the joints of the spine), cisternography (radiological study of CSF flow patterns), CAT scan (Computerized Axial Tomography as a method of resolution of a series of x-ray pictures into a "cross-section" of the body or part of the body in which a contrast agent may be employed), NMR scan or MRI (Magnetic Resonance Imaging as a computerized method of resolution of a series of radio-frequency scans of tissues into a "cross-section" of the body or body part, which visualizes in a tissue-specific manner the composition of areas rather than density as in the CAT scan).

Of specific interest are diagnostic agents that employ technecium (e.g., used in 85% of all medical diagnostic scans, easily forms metal-electron donor complexes or chelates in the presence of a reducing agent, such as electronegative chelating groups illustrated by SH thiols, CO₂- carboxylates, NH amines, PO₄- phosphate, CNOH oximes, OH hydroxyls, **P** phosphines, and NC isonitriles, exhibits good properties for imaging with a gamma camera, and possesses a short half-life of 6 hours that is adequate to synthesize chelate, determine purity, administer and image with a minimum radiation exposure).

Illustrative chelated agents include technecium tagged agents such as technecium albumin (e.g., heart imaging to determine wall motion and ejection fraction, CAD, bypass surgery, heart failure, pre- and post transplant, cardiomyopathy and damage from cardiotoxins (doxirubicin)), technecium albumin aggregate (e.g., pulmonary microcirculation imaging to determine occlusions due to emboli), technecium albumin colloid (e.g., imaging to determine perfusion and clearance rate of the colloid by the reticuloendothelial cells of the liver and spleen, used in cases of abdominal trauma, tumor metastisis, and liver dysfunction such as in cirrhosis), technecium biscisate (e.g., imaging to determine brain perfusion in stroke and lesion determination), technecium disofenine (e.g., imaging of the liver after hepatocytes take up the product followed by excretion into the gall bladder and common bile duct and finally the duodenum, separating acute from chronic cholecystitis (acute - the cystic duct is blocked preventing bile from getting to the gall bladder), technecium exametazine (e.g., brain imaging agent to determine brain death in life support patients, localize seizure foci, dementia, strokes, as well as radiolabeling of leukocytes to located intra-abdominal infections and inflammatory bowel disease), technecium medronate (e.g., imaging of the skeletal system, including scanning for cancer metastasis to bone in breast and prostate cancer, osteomyelitis, Paget's disease, fracture, stress fracture diagnosis), technecium mertiatide (e.g., imaging of kidney function and urine outflow), technecium gluceptate (e.g., radiolabeling of monoclonal antibodies), technecium pentetate (e.g., imaging of the brain for brain tumors and death, renal studies and glomerular filtration rates), technecium pyrophosphate (e.g., heart imaging to determine diagnosis of recent MI with normal cardiac enzymes), technecium labeled red blood cells (e.g., imaging in cardiac studies, localize pre-operatively the site of active lower GI bleeding, heat wrinkled cells are used for spleenic tissue damage diagnosis), technecium sestammibi (Cardiolite®) (e.g., myocardial perfusion imaging, pre-operative localization of parathyroid adenoma and early breast cancer diagnosis), technecium succimer (e.g., determination of functional renal parenchyma in cases of trauma, cysts and scarring), technecium sodium pertechnate TcO4-Na+ (e.g., similar in size and charge to I- and concentrated in thyroid, salivary glands, kidney, stomach and choroid plexus in the brain (blood-brain barrier) for thyroid scans), technecium sulfur colloid (e.g., imaging of bone, liver, spleen to determine reticuloendothelial cell function, primary agent used in determining GI emptying time and GER (gastroesophegeal reflux)), technecium tetrofosmin (Myoview®) (e.g., myocardial perfusion imaging), and technetium-labeled anti-CD 15 monoclonal antibody which selectively binds to neutrophils at the site of infection (e.g., 99mTc Fanolesomab (NeutroSpec®) for detecting/imaging appendicitis, thereby allowing a physician to view a specific functional view of the infection site in less than an hour with the use of a gamma camera, and also for osteomyelitis, fever of unknown origin, postsurgical abscess, IBD and pulmonary imaging).

Other radiolabel generators in addition to technecium include complexes of strontium-yttrium, zing-copper, germanium-gallium, strontium-rubidium, gallium citrate (e.g., imaging to localized inflammation and infection sites), 18F-2-fluoro-2-deoxy-D-glucose (e.g., PET scanning (positron emission tomography) for determining metabolic rate: brain, heart and cancer management (neoplasms have a high glycolytic rate) etc.), iodine radiolabels (e.g., iobenguane sulfate ¹³¹I for imaging and locating functional neurobiastomas and pheochromocytomas; sodium ¹²³I for thyroid imaging; sodium ¹³¹I for total thyroidectomy and treatment of functional thyroid cancer metastatic carcinoma), indium radiolabels (e.g., utilized to radiolabel monoclonal antibodies and peptides via bifunctional chelating agents; such as indium chloride which behaves similar to Fe⁺³ for imaging of tumors, bone marrow, and abscesses (white blood cell labeling); indium satumomabpendetide for labeling of monoclonal antibodies; indium oxine (8-hydroxyquinoline) for replacing gallium radiolabels due to better specificity and better image quality, labeling of platelets and leukocytes for inflection localization and for platelet studies (thrombosis location, life span) and kidney transplantation; indium pentetate for imaging of the spinal canal and CSF spaces in the brain; indium pentreotide for whole body imaging for the diagnosis of somatostatin receptor rich neuroendocrine tumors and metastasis), thallium radiolabels (e.g., thallium chloride for cardiac imaging of viable myocardium which is similar uptake into tissue as seen with K+), and xenon gas - ¹³³Xn - by inhalation and lung scans to localize obstructed regions).

Additional diagnostic agents include radiological contrast agents such as the iodine based compounds (e.g., diatrizoate meglumine, distrizoate sodium, iopanoic acid, tryopanoate sodium, ipdoate sodium, iothalamate meglumine, iodipamide meglumine, iohexol, iopamidol, ioversol, iodixanol, isosulfan blue, pentetreotide), MRI contrast agents (e.g., gadolinium chelated compounds such as gadopentetate dimeglumin, gadoteridol, ferummoxsil, ferumoxides, mangofodipir trisodium), and ultrasound contrast agents (e.g., perflexane - n-perfluorohexane gas, and 1,2-dimyristoyl-sn-glycero-3-phosphocholine (DMPC)).

Suitable encapsulated compounds include, but are not limited to, hemoglobin, a protein, an enzyme, an immunoglobulin, a peptide, an oligonucleotide, or a nucleic acid. Encapsulated enzymes that can be used with the vesicles described herein include, but are not limited to, alkaline phosphatase, D-amino acid oxidase, 6- aminolevulinate dehydratase, α-amylase, amyloglucosidase, ascorbate oxidase, asparaginase, butyrylcholinesterase, catalase, carbonic anhydrase, chloroperoxidase, cholesterol esterase, chymotrypsin, a chymotrypsin, cyprosin, dextranase, DNA photolyase, DNA- (apurinic or apyrimidinic site) lyase, DNA polymerase, DNase I, elastase, enzyme extract from Lactobacillus helveticus, FLAVOURZYME 9, β-fructofuranosidase, β- galactosidase, β-glucosidase, glucocerbroside-β-glucosidase, glucose oxidase, glucose oxidase-insulin, glucose-6-phosphate-dehydrogenase, β-glucuronidase, hexokinase, β- lactamase, lipase from Chromobacterium viscosum, luciferase, lysozyme, neutrases, pepsin A, peroxidase, peroxidase+glucose oxidase, phosphatase, phospatase from Citrobacter, phospholipase A2, phospholipase C, phospholipase D, phosphorylase, phosphotriesterase, t- plasminogen activator, polynucleotide phosphorylase, proteinase, proteinase K, Qo replicase/MDV-I RNA, ribonuclease A, rulactine, Sn-glycerol-3-phosphate O- acyltransferase, sphingomylinase, streptokinase, superoxide dismutase, superoxide dismutase+catalase, trypsin, tyrosinase, urease, and urate oxidase.

Encapsulated nucleic acids and nucleic acid sequences that can be used with the vesicles described herein include, but are not limited to, nucleic acids isolated from viral, prokaryotic, eukaryotic, bacterial, plant, animal, mammal, and human sources. Other kinds of nucleic acids include, but are not limited to, antisense oligonucleotides, RNAi agents, aptamers, primers, plasmids, catalytic nucleic acid molecules, e. g., ribozymes, triplex forming molecule, and antiangiogenic oligonucleotides. Further examples include recombinant DNA molecules that are incorporated into a vector, such as an autonomously replicating plasmid or virus, or that insert into the genomic DNA of a prokaryote or eukaryote, e. g., as a transgene or as a modified gene or DNA fragment introduced into the genome by homologous recombination or site-specific recombination, or that exist as separate molecules, e. g., a cDNA or a genomic or cDNA fragment produced by PCR, restriction endonuclease digestion, or chemical or *in vitro* synthesis. Useful nucleic acids can also include any recombinant DNA molecule that encodes any naturally-or non-naturally occurring polypeptide. Other nucleic acids include RNA, e.g., an mRNA molecule that is encoded by an isolated DNA molecule, or that is chemically synthesized, a short interfering RNA molecule (i.e., an RNAi agent), etc. The terms "nucleic acid," "nucleotide," "oligonucleotide," "DNA," and "RNA" are known to one of ordinary skill in the art. Definitions of these terms are also found in the World Intellectual Property Organization (WIPO) Handbook on Industrial Property Information and Documentation, Standard ST. 25: Standard for the Presentation of Nucleotide and Amino Acid Sequence Listings in Patent Applications (1998), including Tables 1 through 6 in Appendix 2, incorporated herein by reference (hereinafter "WIPO Standard ST. 25 (1998)"). In certain aspects described herein, the terms" nucleic acid, ""DNA," and "RNA" include derivatives and biologically functional equivalents. In certain aspects described herein, the terms "nucleic acid," "nucleic acid sequence," and "oligonucleotide" are used interchangeably. These terms refer to a polymer of nucleotides (dinucleotide and greater), including polymers of 2 to about 100 nucleotides in length, including polymers of about 101 to about 1,000 nucleotides in length, including polymers of about 1,001 to about 10,000 nucleotides in length, and including polymers of more than 10,000 nucleotides in length.

In another aspect, amino acids and amino acid sequences such as proteins and peptides can be used with the vesicles described herein. Suitable proteins can include, but are not limited to, insulin and pepsin. Also, encapsulated proteins and peptides can include large molecular weight therapeutic peptides and proteins such as, for example, GLP-1, CCK, antimicrobial peptides, and antiangiogenics. Proteins, such as insulin, that can be incorporated into liposomes can be found in Kim et al., Int. J. Pharm., 180,75-81, 1999, which is incorporated herein by reference for its teachings of encapsulated proteins and peptides. The terms "amino acid" and "amino acid sequence" are known to one of ordinary skill in the art. Definitions of these terms are also found in the WIPO Standard ST. 25 (1998). In certain aspects described herein, the terms "amino acid" and "amino acid sequence" include derivatives, mimetics, and analogues including D-and L-amino acids which cannot be specifically defined in WIPO Standard ST. 25 (1998). The terms "peptide" and "amino acid sequence "are used interchangeably herein and refer to any polymer of amino acids (dipeptide or greater) typically linked through peptide bonds. The terms "peptide" and "amino acid sequence" include oligopeptides, protein fragments, analogues, nuteins, and the like.

### VESICLE COMPRISING COMPOSITIONS

Aspects of the invention further include compositions that comprise a plurality of vesicles of the invention, e.g., as described above. The concentration of vesicle in a given composition may vary, and may range from about 5 to about 100, such as from about 90 to about 100%. In certain embodiments, the compositions are characterized by exhibiting low size polydispersity with respect to vesicles present in the composition. By "low size polydispersity" is meant that the vesicles in the composition have diameters that differ from each other by about 10 % or less, such as by about 5% or less. As reviewed above, the vesicles may include an active agent, e.g., a diagnostic or therapeutic agent.

In certain embodiments, the compositions are pharmaceutical compositions. A variety of suitable methods of administering a formulation of the present invention to a subject or host, e.g., patient, in need thereof, are available. Although more than one route can be used to administer a particular formulation, a particular route can provide a more immediate and more effective reaction than another route. Any convenient pharmaceutically acceptable excipients may be employed. The choice of excipient will be determined in part by the particular compound, as well as by the particular method used to administer the composition. Accordingly, there is a wide variety of suitable formulations of the pharmaceutical composition of the present invention. The following methods and excipients are merely exemplary and are in no way limiting.

Formulations suitable for oral administration include, but are not limited to: (a) liquid solutions, such as an effective amount of the compound dissolved in diluents, such as water, saline, or orange juice; (b) capsules, sachets or tablets, each containing a predetermined amount of the active ingredient, as solids or granules; (c) suspensions in an appropriate liquid; and (d) suitable emulsions. Tablet forms can include one or more of lactose, mannitol, corn starch, potato starch, microcrystalline cellulose, acacia, gelatin, colloidal silicon dioxide, croscarmellose sodium, talc, magnesium stearate, stearic acid, and other excipients, colorants, diluents, buffering agents, moistening agents, preservatives, flavoring agents, and pharmacologically compatible excipients. Lozenge forms can comprise the active ingredient in a flavor, usually sucrose and acacia or tragacanth, as well as pastilles comprising the active ingredient in an inert base, such as gelatin and glycerin, or sucrose and acacia, emulsions, gels, and the like containing, in addition to the active ingredient, such excipients as are known in the art.

The subject formulations of the present invention can be made into aerosol formulations to be administered via inhalation. These aerosol formulations can be placed into pressurized acceptable propellants, such as dichlorodifluoromethane, propane, nitrogen, and the like. They may also be formulated as pharmaceuticals for non-pressured preparations such as for use in a nebulizer or an atomizer.

Formulations suitable for parenteral administration include aqueous and non-aqueous, isotonic sterile injection solutions, which can contain anti-oxidants, buffers, bacteriostats, and solutes that render the formulation isotonic with the blood of the intended recipient, and aqueous and non-aqueous sterile suspensions that can include suspending agents, solubilizers, thickening agents, stabilizers, and preservatives. The formulations can be presented in unit-dose or multi-dose sealed containers, such as ampules and vials, and can be stored in a freeze-dried (lyophilized) condition requiring only the addition of the sterile liquid excipient, for example, water, for injections, immediately prior to use. Extemporaneous injection solutions and suspensions can be prepared from sterile powders, granules, and tablets of the kind previously described.

Formulations suitable for topical administration may be presented as creams, gels, pastes, or foams, containing, in addition to the active ingredient, such carriers as are known in the art to be appropriate.

Suppository formulations are also provided by mixing with a variety of bases such as emulsifying bases or water-soluble bases. Formulations suitable for vaginal administration may be presented as pessaries, tampons, creams, gels, pastes, foams.

Unit dosage forms for oral or rectal administration such as syrups, elixirs, and suspensions may be provided wherein each dosage unit, for example, teaspoonful, tablespoonful, tablet or suppository, contains a predetermined amount of the composition containing one or more inhibitors. Similarly, unit dosage forms for injection or intravenous administration may comprise the inhibitor(s) in a composition as a solution in sterile water, normal saline or another pharmaceutically acceptable carrier.

The term "unit dosage form," as used herein, refers to physically discrete units suitable as unitary dosages for human and animal subjects, each unit containing a predetermined quantity of compounds of the present invention calculated in an amount sufficient to produce the desired effect in association with a pharmaceutically acceptable diluent, carrier or vehicle. The specifications for the novel unit dosage forms of the present invention depend on the particular compound employed and the effect to be achieved, and the pharmacodynamics associated with each compound in the host.

Those of skill in the art will readily appreciate that dose levels can vary as a function of the specific compound, the nature of the delivery vehicle, and the like. Preferred dosages for a given compound are readily determinable by those of skill in the art by a variety of means.

The dose administered to an animal, particularly a human, in the context of the present invention should be sufficient to effect a prophylactic or therapeutic response in the animal over a reasonable time frame. One skilled in the art will recognize that dosage will depend on a variety of factors including the strength of the particular compound employed, the condition of the animal, and the body weight of the animal, as well as the severity of the illness and the stage of the disease. The size of the dose will also be determined by the existence, nature, and extent of any adverse side-effects that might accompany the administration of a particular compound.

### METHODS OF MAKING

Aspects of the invention further induce methods for preparing vesicles as described above. Generally, the methods include providing a mixture of fluid polar medium comprising self -assembling block copolymers that include a first hydrophobic domain and a second hydrophilic intracellular transduction domain, as described above, and then maintaining the mixture under conditions sufficient to produce the Vesicles. In certain embodiments, the mixture includes a sufficient amount of the copolymer(s) present in an aqueous medium, where the aqueous medium may further include one or more active agents, e.g., as described above. The amount of copolymer present in the mixture may vary. In certain embodiments the amount of copolymer present in the mixture ranges from about 0.1 % weight/volume (w/v) to about 5%, such as from about 0.5 to about 3% and including from about 1 to about 2%. If present, the concentration of active agent, e.g., water-soluble active agent, may vary. In certain embodiments the concentration of active agent present in the mixture ranges from about 1 nM to about 100 mM, such as from about 1 microM to about 100 microM. This concentration will also depend on the potency of the active agent.

The provided mixture is maintained under conditions sufficient to produce the desired vesicles, e.g., under self-assembling reaction conditions. Suitable conditions are those conditions sufficient to provide for the self-assembly or association of the disparate copolymer building blocks into a vesicle. In certain embodiments, the conditions under which self-assembly of the copolymers occurs are physiologic conditions or other laboratory conditions under which the individual component proteins would be stable. In certain embodiments, the conditions comprise an aqueous medium having a pH ranging from about 4 to 10, such as from about 6 to 8, where the temperature ranges from about 4ºC to about 100ºC.

Where desired, the product composition that includes a plurality of vesicles may be filtered or otherwise sorted to produce a composition having low polydispersity with respect to the size of the vesicles in the composition. Further details regarding embodiments of methods of making the vesicles may be found in the Experimental section, below. Furthermore, the protocols described in (Holowka, E. P., Pochan, D. J., Deming, T. J. Charged Polypeptide Vesicles with Controllable Diameter, J. Amer. Chem. Soc. 127, 12423-12428 (2005)), may be employed, where the copolymers employed in this Holowka et al., reference are substituted with the copolymers employed in the present invention, e.g., as described above.

### UTILITY

The disclosed vesicles, e.g., which may include encapsulated compounds such as therapeutic or diagnostic agents, have many uses. In one aspect, disclosed herein is a method of treating or preventing a disease in a subject comprising administering to the subject vesicles containing an encapsulated compound (i.e., active agent) as discussed above. The selection of the encapsulated compound is based on the particular target disease in a subject.

The dosage or amount of vesicles administered to a given subject should be large enough to produce the desired effect in which delivery occurs. The dosage should not be so large as to cause adverse side effects, such as unwanted cross-reactions, anaphylactic reactions, and the like. Generally, the dosage will vary with the age, condition, sex and extent of the disease in the subject and can be determined by one of skill in the art. The dosage can be adjusted by the individual physician in the event of any counterindications. The dose, schedule of doses and route of administration can be varied, whether oral, nasal, vaginal, rectal, extraocular, intramuscular, intracutaneous, subcutaneous, intravenous, intratumoral, intrapleural, intraperitoneal or other practical routes of administration to avoid adverse reactions yet still achieve delivery.

The vesicles described herein can be used therapeutically in combination with a pharmaceutically acceptable carrier to produce a pharmaceutical composition, such as the compositions described above.

In one aspect, the vesicles described herein are administered to a subject such as a human or an animal including, but not limited to, a rodent, dog, cat, horse, bovine, ovine, or non-human primate and the like, that is in need of alleviation or amelioration from a recognized medical condition. The vesicles can be administered to the subject in a number of ways depending on whether local or systemic treatment is desired, and on the area to be treated. Administration can be topically (including ophthalmically, vaginally, rectally, intranasally), orally, by inhalation, or parenterally, for example by intravenous drip, subcutaneous, intraperitoneal or intramuscular injection. The vesicles described herein can be administered intravenously, intraperitoneally, intramuscularly, subcutaneously, intratumoral, intracavity, or transdermally.

In another aspect, disclosed herein are methods for screening a vesicle-encapsulated compound for an activity by (a) measuring a known activity or pharmacological activity of the vesicle-encapsulated compound; and (b) measuring the same activity or pharmacological activity of the corresponding unencapsulated compound.

The activities for which the vesicle-encapsulated compound can be screened can include any activity associated with a biologically active compound. The following is a partial list of the many activities that can be determined in the present screening method: 1. Receptor agonist/antagonist activity: A compendia of examples of specific screens for measuring these activities can be found in: "The RBI Handbook of Receptor Classification and Signal Transduction" K. J. Watling, J. W. Kebebian, J. L. Neumeyer, eds. Research Biochemicals International, Natick, MA, 1995, and references therein. Methods of analysis can be found in: T. Kenakin "Pharmacologic Analysis of Drug-Receptor Interactions"2nd Ed. Raven Press, New York, 1993, and references therein; Enzyme inhibition: A compendia of examples of specific screens for measuring these activities can be found in: H. Zollner "Handbook of Enzyme Inhibitors", 2nd Ed. VCH Weinheim, FRG, 1989, and references therein; Central nervous system, autonomic nervous system (cardiovascular and gastrointestinal tract), antihistaminic, anti-inflammatory, anaesthetic, cytotoxic, and antifertility activities: A compendia of examples of specific screens for measuring these activities can be found in: E. B. Thompson, "Drug Bioscreening: Drug Evaluation Techniques in Pharmacology, "VCH Publishers, New York, 1990, and references therein; Anticancer activities: A compendia of examples of specific screens for measuring these activities can be found in: I. J. Fidler and R. J. White"Design of Models for Testing Cancer Therapeutic Agents, "Van Nostrand Reinhold Company, New York, 1982, and references therein; Antibiotic and antiviral (especially anti-HIV) activities: A compendia of examples of specific screens for measuring these activities can be found in:"Antibiotics in Laboratory Medicine, "3rd Ed. , V. Lorian, ed. Williams and Wilkens, Baltimore, 1991, and references therein. A compendia of anti-HIV screens for measuring these activities can be found in :"HIV Volume 2: Biochemistry, Molecular Biology and Drug Discovery," J. Karn, ed. , IRL Press, Oxford, 1995, and references therein; Immunomodulatory activity: A compendia of examples of specific screens for measuring these activities can be found in: V. St. Georgiev, "Immunomodulatory Activity of Small Peptides, "Trends Pharm. Sci. 11, 373-378 1990; Pharmacokinetic properties: The pharmacological activities assayed in the screening method include half- life, solubility, or stability, among others. For example, methods of analysis and measurement of pharmacokinetic properties can be found in: J. -P. Labaune "Handbook of Pharmacokinetics : Toxicity Assessment of Chemicals, "Ellis Horwood Ltd., Chichester, 1989, and references therein; Oxygen Carrying Capacity The functional capacity of compounds such as hemoglobin is assessed both in vitro as well as in vivo. Methods of analysis are described in: Reiss, Chem. Rev., 101, 2797,2001 and references therein; Rabinovici et al., Circulatory Shock, 32,1, 1990; Methods Enzymol., Vols. 231 & 232; Proctor, J. Trauma, 54, S106, 2003 and references therein.

In the screening method, the vesicle can be any of the vesicles described herein. Also, the encapsulated compound, which corresponds to the unencapsulated compound, can be any of the encapsulated compounds described herein.

Thus, in the screening method contemplated herein, any vesicle with an encapsulated compound, i.e., vesicle-encapsulated compound, can be compared to the corresponding unencapsulated compound having a known activity to determine whether or not it has the same or similar activity at the same or different level. Depending on the specifics of how the measuring step is carried out, the present screening method can also be used to detect an activity exhibited by the unencapsulated compound of step b) that differs qualitatively from the activity of the encapsulated compound of step a).

Also, the screening method can be used to detect and measure differences in the same or similar activity. Thus, the screening methods described herein take into account the situation in which the differences of the vesicle-encapsulated compound significantly alter the biological activity of the unencapsulated compound.

### SYSTEMS & KITS

Systems and kits with formulations used in the subject methods, are provided. Conveniently, the formulations may be provided in a unit dosage format, which formats are known in the art.

In such systems and kits, in addition to the containers containing the formulation(s), e.g. unit doses, is an informational package insert describing the use of the subject formulations in the methods of the subject invention, e.g., instructions for using the subject unit doses to treat cellular proliferative disease conditions.

These instructions may be present in the subject systems and kits in a variety of forms, one or more of which may be present in the kit. One form in which these instructions may be present is as printed information on a suitable medium or substrate, e.g., a piece or pieces of paper on which the information is printed, in the packaging of the kit, in a package insert, etc. Yet another means would be a computer readable medium, e.g., diskette, CD, etc., on which the information has been recorded. Yet another means that may be present is a website address which may be used via the internet to access the information at a removed site. Any convenient means may be present in the kits.

The following examples are offered by way of illustration and not by way of limitation.

### EXAMPLES

### I. Materials and Methods

**A. Synthesis.** All block copolypeptides were synthesized using Co(PMe₃)₄ initiator (Deming, T.J. "Cobalt and iron initiators for the controlled polymerization of alpha-amino acid-N-carboxyanhydrides," Macromolecules 32, 4500-4502 (1999)), and were purified and then characterized using size exclusion chromatography, ¹H and ¹³C NMR, and IR spectroscopy according to literature procedures (Holowka et al., "Charged Polypeptide Vesicles with Controllable Diameter, J. Amer. Chem. Soc. 127, 12423-12428 (2005)). K₆₀L₂₀ was prepared as previously described. Isolated yields of the final copolymers ranged between 75% and 98%. Copolypeptide compositions determined using GPC/LS were found to be within 5% of predicted values. Chain lengths of the copolymers were found to be within 8% of predicted lengths with CLD (weight average length/number average length) ranging between 1.1 and 1.3.

**Poly(di-*N*-benzyloxycarbonyl-L-arginine_{0.9}-*random*-*N-*benzyloxycarbonyl-L-lysine_{0.1})₆₀**-***block*-Poly(L-leucine)₂₄, [(Z₂-R)_{0.9}/(Z-K)_{0.1}]₆₀L₂₀** In a nitrogen atmosphere dry box, Z₂-Arg NCA (200 mg, 0.42 mmol) and N_{ε}-benzyloxycarbonyl-L-lysine-N-carboxyanhydride (Z-Lys NCA) (12 mg, 0.042 mmol) were dissolved in THF (8 mL) and placed in a 20 mL scintillation vial with stir bar. A Co(PMe₃)₄ initiator solution (100 µL of a 0.047 µM solution in THF) was then added to the vial via syringe. The vial was then sealed and allowed to stir in the dry box for 4 hours at 25°C. After 4 hours, an aliquot (50 µL) was removed and diluted to a concentration of 5 mg/mL in DMF containing 0.1 M LiBr for GPC/LS analysis (Mₙ = 26,740; M_{w}/Mₙ = 1.17). The remainder of he aliquot was analyzed by FTIR to confirm that all the Z₂-Arg NCA and Z-Lys NCA had been consumed. In the dry box, L-leucine-N-carboxyanhydride (Leu NCA) (35 mg, 0.23 mmol) was dissolved in THF (0.7 mL) and then added to the reaction vial. The polymerization was allowed to continue with stirring at 25 °C in the dry box for another 3 hours. After 3 hours, an aliquot (50 µL) was removed and diluted to a concentration of 5 mg/mL in DMF containing 0.1 M LiBr for GPC/LS analysis (Mₙ = 29,230; M_{w}/Mₙ = 1.27). The remainder of the aliquot was analyzed by FTIR to confirm that all the Leu NCA had been consumed. Outside of the dry box, the copolypeptide was then precipitated by adding the THF solution to methanol (50 mL), and then isolated by centrifugation. The polymer pellet was then soaked in methanol (50 mL) for 2 hours before a second centrifugation to give the protected copolymer, after drying under vacuum for several hours, as a white powder (165 mg, 91 % yield). The average composition of the copolymer as determined by GPC/LS was [(Z₂-R)_{0.9}/(Z-K)_{0.1}]₆₃L₂₁.

**Poly(L-arginine_{0.9}-*random*-L-lysine_{0.1})₆₀-*block*-Poly(L-leucine)₂₀: (R_{0.9}/K_{0.1})₆₀L₂₀** A 100 mL round-bottom flask was charged with [(Z₂-R)_{0.9}-(Z-K)_{0.1}]₆₀L₂₀ (155 mg) and TFA (8 mL). The flask was placed in an ice bath and allowed to stir for 15 minutes, which allowed the polymer to dissolve and the contents of the flask to cool to 0 °C, At this point, HBr (1.8 mL of a 33 % solution in HOAc, 10 equivalents) was added dropwise and the solution was then allowed to stir in the ice bath for 1 hour. After this time, diethyl ether (20 mL) was added in order to precipitate the product. The mixture was centrifuged to isolate the solid precipitate, and the product was subsequently washed with diethyl ether (20 mL) several times to yield a white solid. After drying the sample in air, it was resuspended in pyrogen free water (10 mL), LiBr (150 mg) was added, and the solution was placed in a dialysis bag (MWCO = 2000 Da). The sample was dialyzed against EDTA (3 mM in pyrogen free water) for one day in order to remove residual cobalt initiator, and then for 2 additional days against pyrogen free water (water changed every 8 hours). Pyrogen free water was obtained from a Millipore Milli-Q Biocel A10 purification unit. After dialysis, the sample was lyophilized to give the product as a white fluffy powder (54 mg, 94 % yield).

**FITC Functionalization of (R_{0.9}/K_{0.1})₆₀L₂₀ to give R₆₀L₂₀** Fluorescent tagging of lysine ε-amine groups was done using fluorescein isothiocyanate (FITC) dissolved in DMSO (10 mg/mL). R_{0.9}/K_{0.1})₆₀L₂₀ powder (100 mg) was dissolved in a mixture of aqueous NaHCO₃(10 mL, 0.2 M) and THF (10 mL). To the polypeptide solution, 5.4 equivalents of FITC per chain (corresponding to 54% of the available lysine amines) was added and mixture was stirred for 16h. For purification, samples were dialyzed (MWCO = 8000 Da) in the dark for 4 days with pyrogen free water changed every 12 hours. The functionalized polymer was isolated by lyophilization to give a slightly yellow powder (103 mg).

**Preparation of R₆₀L₂₀ Vesicle Assemblies in Water** R₆₀L₂₀ powder was dispersed in THF to give a 1 % (w/v) suspension, which was then placed in a bath sonicator for 30-45 minutes until the copolypeptide was evenly dispersed and no large particulates were observed. A stir bar was added followed by dropwise addition of an equal volume of pyrogen free water under constant stirring. The stir bar was then removed and the mixture was placed in a bath sonicator for 30 minutes, after which the mixture was placed in a dialysis bag (MWCO = 2000 Da) and dialyzed against pyrogen free water for 24 h. The water was changed every hour for the first 5 hours, and subsequently every 6 hours. The resulting vesicle suspensions were extruded using an Avanti Mini-Extruder. Extrusions were performed using different pore size Whatman Nucleopore Track-Etch polycarbonate (PC) membranes (1.0 µm, 0.4 µm, 0.2 µm, 0.1 µm, and 0.05 µm) at room temperature. The PC membranes were soaked in pyrogen free water for 10 minutes prior to extrusion. After two passes through the mini-extruder, the resulting suspensions were analyzed using DIC optical microscopy and DLS. Vesicles of 100 nm average diameter were used for all the cell studies.

**Dextran Encapsulation by Vesicle Extrusion** A 100 µM suspension of R₆₀L₂₀ vesicles in pyrogen free water was prepared as described above. To this suspension was added an equal volume of Texas Red labeled dextran (3000 Da, 0.250 mg/mL) in deionized water to give a final dextran concentration of 0.125 mg/mL. This suspension was then extruded through a 0.1 µm PC membrane 4 times. The resulting sample was then dialyzed (MWCO = 6000-8000 Da) against pyrogen free water for 12 hours to remove dextran that had not been encapsulated by the vesicles. The amount of encapsulated dextran was then quantified spectrophotometrically according to published procedures.

**Chloroform/Water Partitioning** Copolypeptide vesicle suspensions were prepared at 2 (w/v) % and diluted in test tubes to 1 (w/v) % with aqueous buffer (0.5 mL; 10 mM NaH₂PO₄, 100 mM NaCl, pH 7.4). Chloroform-lipid solutions (0.5 mL) were prepared (10 mM of either EYPG or EYPC) and were layered with the aqueous suspensions in the test tubes. Care was taken to minimize perturbation to the aqueous layer. The two-phase systems all initially showed a turbid water phase and a clear chloroform phase. The test tubes were then centrifuged at 3,000 rpm for 30 minutes. The samples were then removed and the EYPG sample was found to have a clear water layer and turbid chloroform layer for EYPG, while the EYPC sample had not changed. Subsequent laser scanning confocal microscopy of the samples revealed the presence of vesicles within the chloroform layer for EYPG, with no visible population within the aqueous layer. The opposite was found for the sample with EYPC. For the EYPG sample, the chloroform layer was removed and added to another vial containing solution of NaHSO₄ (10 mM) in water. A stir bar was added and the contents of the vial were gently stirred for 1 hour. Confocal microscopy of the sample revealed the presence of vesicles in the aqueous phase with a negligible population remaining in the chloroform layer.

**B. Materials.** Phosphate-buffered saline (PBS), penicillin-streptomycin, a 1:1 mixture of Dulbecco's modified Eagle's medium and Ham's F12 medium (DMEM/F12), and MDCB 131 medium were purchased from Invitrogen (Carlsbad, CA). Fetal bovine serum (FBS) was obtained from Hyclone (Logan, UT), while L-glutamine and epidermal growth factor (EGF) were purchased from Becton-Dickinson (Franklin Lakes, NJ). All other cell culture reagents were purchased from Sigma (St. Louis, MO). The T84 cell line was obtained from the American Type Culture Collection (ATCC) (Manassas, VA), while the HULEC-5A cell line was generously provided by the Centers for Disease Control and Prevention (Atlanta, GA). Cell counting was performed with the Coulter counter, and the isotonic solution for the Coulter counter was purchased from Beckman Coulter (Fullerton, CA). Coverslip-bottom glass dishes were obtained from BD Biosciences (San Jose, CA). The MTT cell survival assay kit was purchased from Chemicon International (Temecula, CA).

**C. Laser Scanning Confocal Microscopy.** Confocal fluorescence images of aqueous and organic phase vesicles at 1 %(w/v) were taken on a Leica TCS-SP MP Confocal and Multiphoton Inverted Microscope (Heidelberg, Germany) equipped with an argon laser (488 nm blue excitation: JDS Uniphase) and a 561 nm (green) diode laser (DPSS: Melles Griot) and a two photon laser setup consisting of a Spectra-Physics Millenia X 532 nm green diode pump laser and a Tsunami Ti-Sapphire picosecond pulsed infrared laser tuned at 768 nm for UV excitation.

**D. Transmission Electron Microscopy.** R₆₀L₂₀ vesicle suspensions (0.1 % (w/v)) were extruded separately through through 0.05, 0.1, 0.2, and 0.4 µm polycarbonate (PC) membranes. One drop of each respective sample was placed on a 200 mesh Formvar coated copper grid (Ted Pella) and allowed to stand on the grid for 90 seconds. Filter paper was then used to wick away residual sample and liquid. One drop of 1 % (w/v) aqueous uranyl acetate (negative stain) was then placed on the grid, allowed to stand for 20 seconds, and subsequently removed by washing the grid with drops of pyrogen free water and wicking away excess liquid with filter paper. The resulting samples were imaged using a JEOL 100 CX transmission electron microscope at 80 keV and ambient temperature.

**D. Cell Culture.** The T84 cell line is an epithelial tumor cell line derived from a human lung metastasis of a colon carcinoma. These cells were maintained in DMEM/F12 supplemented with 13.5 mM sodium bicarbonate, 5% FBS, 100 units/mL penicillin, and 100 µg/mL streptomycin at a pH of 7.4 in a 37 °C humidified atmosphere with 5% CO₂. The HULEC-5A cell line is a human endothelial cell line that was derived from lung microvasculature and transformed with an SV-40 large T antigen. These cells were cultured in MDCB 131 medium containing 14 mM sodium bicarbonate, 10% FBS, 100 units/mL penicillin, 100 µg/mL streptomycin, 10 mM L-glutamine, 10 ng/mL EGF, and 1 µg/mL hydrocortisone at a pH of 7.4 in a 37°C humidified atmosphere with 5% CO₂.

**E. Cellular uptake of polypeptide vesicles.** The T84 and HULEC-5A cells were seeded at densities of 1x10⁵ and 5x10⁴ cells/cm², respectively, on coverslip-bottom glass dishes 12-14 hours prior to the start of the experiment. The seeding densities were slightly different due to differences in the cell sizes and proliferation rates. Note that the seeding medium was the same as the cell culturing medium. Before the addition of polypeptide vesicles, the seeding medium was aspirated off, and the cells were incubated for 5 hours with an incubation medium containing a 100 µM polypeptide vesicle suspension, or Texas Red labeled dextran (3000 Da, 1 µM) in deionized water as a control, in a 37°C humidified atmosphere inside an air incubator. The incubation medium was the same as the cell culturing medium except for the absence of FBS and the presence of 20 mM HEPES instead of sodium bicarbonate. FBS was initially excluded from the incubation medium since the net-negatively charged proteins in FBS had the potential to interfere with polypeptide-cell interactions. Subsequent studies were also performed in the presence of FBS and at 0 °C. After the incubation period, the cells were washed with PBS, and placed in contact with the seeding medium for 5 minutes. This medium was then aspirated to ensure that all observed fluorescence was derived from internalized vesicles. Finally, the cells were placed in contact with PBS and visualized using confocal microscopy.

### II. Results

R₆₀L₂₀ block copolypeptides were prepared using established procedures (Deming, T.J. Facile synthesis of block copolypeptides of defined architecture. Nature 390, 386-389 (1997)). These samples showed physical properties similar to the K₆₀L₂₀ materials and were found to form micron-sized vesicles in aqueous solution (Figure 1a,b)(Holowka, E. P., Pochan, D. J., Deming, T. J. Charged Polypeptide Vesicles with Controllable Diameter, J. Amer. Chem. Soc. 127, 12423-12428 (2005)). These vesicles were able to entrap water soluble species, such as dextran (Figure 1 c), and could be extruded through polycarbonate filters to yield stable, low polydispersity vesicles of controllable diameter down to 50 nm (Figure 1d,e) >Discher, B.M., Hammer, D.A., Bates, F.S., Discher, D.E. Polymer vesicles in various media, Curr. Opn. Coll. Interface. Sci. 5, 125-145 (2000)). For facile imaging of the polypeptides, the R₆₀ segments were prepared to contain 10 mole% randomly placed lysine residues that allowed facile attachment of fluorescein dyes via isothiocyanate coupling to the lysine amine groups (Figure 1a). These labeled samples were found to exhibit the same properties as those of the unlabeled, lysine-free samples and for simplicity will be designated as "R₆₀L₂₀" in this paper.

To see if the use of R₆₀ segments would enhance transport across membrane interfaces, we first studied the partitioning of the polypeptide vesicles at bulk water/chloroform interfaces, as has been used previously to evaluate PTD conjugates (Sakai, N., Matile, S. Anion-mediated transfer of polyarginine across liquid and bilayer membranes, J. Amer. Chem. Soc., 125, 14348-14356 (2003); Rothbard, J.B., Jessop, T.C., Lewis, R.S., Murray, B.A., Wender, P.A. Role of membrane potential and hydrogen bonding in the mechanism of translocation of guanidinium-rich peptides into cells, J. Amer. Chem. Soc., 126, 9506-9507 (2004)). In this study, the polypeptide vesicles were prepared in an aqueous phosphate-buffered saline (PBS) buffer, which was then layered onto a solution of lipid in chloroform. The samples were gently mixed and the contents of each phase examined using laser scanning confocal microscopy (LSCM). Similar to PTD conjugates, the R₆₀L₂₀ vesicles were found to remain in the aqueous phase when a neutral zwitterionic lipid, egg yolk phosphatidyl choline (EYPC), was in the chlorofom phase (Figure 2d), yet transferred into the organic phase when an anionic lipid, egg yolk phosphatidyl glycerol (EYPG), was used (Figure 2b) (Sakai et al., supra). The absence of lipid in the chloroform phase (Figure 2a), or the use of K₆₀L₂₀ vesicles (Figure 2e), resulted in no transport of vesicles into the organic layer, attesting to the importance of counterion binding to the arginine residues for transport. Furthermore, R₆₀L₂₀ vesicles loaded with Texas Red labeled dextran (3000 Da) were found to not lose their contents during transport (Figure 2f). When the chloroform-EYPG solution containing the R₆₀L₂₀vesicles was layered with a fresh aqueous phase containing sulfate ions, which bind guanidine residues stronger than phospholipid headgroups, the vesicles were found to migrate back to the aqueous phase, demonstrating the capability for transport in and out of a hydrophobic environment, analogous to membrane transport (Figure 2c) (Sakai et al., supra). The remarkable observation from these studies was that the R₆₀L₂₀ vesicles were found to transport across the interface intact, without vesicle disruption, showing the robust nature of these vesicles and their ability to carry large cargos across interfaces without leakage.

These promising results led us to test the potential of the R₆₀L₂₀ vesicles for intracellular delivery *in vitro.* We examined both epithelial (T84) and endothelial (HULEC-5A) and cell lines because of their relevance in oral and intravenous drug delivery, respectively. Cultures of both cell types were incubated over a time course of 5 hours in serum free media with 100 nm average diameter R₆₀L₂₀ vesicles containing the model cargo Texas Red labeled dextran (3000 Da). Examination of the non-fixed cells using LSCM showed that the vesicles and their contents were rapidly taken up by both cell lines (Figure 3), similar to the uptake observed for smaller oligoarginine PTDs (Rothbard, J.B., Jessop, T.C., Wender, P.A. Adaptive translocation: the role of hydrogen bonding and membrane potential in the uptake of guanidinium-rich transporters into cells, Adv. Drug Deliv. Rev. 57, 495-504 (2005); Wadia, J.S., Dowdy, S.F. Transmembrane delivery of protein and peptide drugs by TAT-mediated transduction in the treatment of cancer, Adv. Drug Deliv. Rev. 57, 579-596 (2005)). Control experiments with fluorescein labeled K₆₀L₂₀ vesicles (Figure 3h), and with unencapsulated Texas Red labeled dextran (see Supplementary Information) both showed minimal cellular uptake, verifying that the polyarginine segments were responsible for vesicle uptake and internalization of their dextran contents. Quantification of fluorescence intensity from the images showed greatly enhanced (up to 16 times) uptake of the encapsulated dextran cargo compared to uptake of free dextran in the presence of unloaded R₆₀L₂₀ vesicles.

Three dimensional reconstructions of the LSCM image slices showed that vesicles as well as their dextran contents were internalized mainly as punctate regions within the cells, and partially co-localized, implying that both the vesicles and their contents enter the cells together (Figure 3e). Incubation of the cell lines with R₆₀L₂₀ vesicles at 0 °C also showed uptake (Figure 3g), and the amount of vesicle uptake was only slightly diminished compared to the incubations at 37 °C, similar to earlier findings with short arginine peptides (Rothbard et al., supra). Vesicle uptake may occur via macropinocytosis, which has been proposed as an uptake mechanism for PTDs (Wadia, J.S., Stan, R.V., Dowdy, S.F. Transducible TAT-HA fusogenic peptide enhances escape of TAT-fusion proteins after lipid raft macropinocytosis, Nature Medicine 10, 310-315 (2004)), and can explain how the relatively large 100 nm vesicles are internalized. The rapid cellular uptake of the R₆₀L₂₀ vesicles shows that, contrary to current thinking (Mitchell, D.J., Kim, D.T., Steinman, L., Fathman, C.G., Rothbard, J.B. Polyarginine enters cells more efficiently than other polycationic homopolymers, J. Peptide Res. 56, 318-325 (2000)), larger polyarginine chains can be effective for intracellular delivery provided that they are correctly presented. In our system, the polyarginine segments are not free to diffuse in solution, but are tethered together in the vesicular self-assembly, which can mask some of the guanidine groups by allowing only the chain-ends to interact with the cell surfaces. Furthermore, since the oligoleucine hydrophobic interactions are stronger than the polyarginine-cell interactions, the vesicles do not disrupt upon cell binding.

Although the R₆₀L₂₀ vesicles were internalized, the potential cytotoxicity of the polyarginine chains needed to be addressed. The toxicity of the polypeptide vesicles was assayed in both T84 and HULEC-5A cells using the MTT cell survival assay, which measures metabolic activity (Mosmann, T. Rapid colorimetric assay for cellular growth and survival: Application to proliferation and cytotoxicity assays, Journal of Immunological Methods 65, 55-63 (1983)). Cells incubated with R₆₀L₂₀ vesicles or R₆₀ homopolymer were found to be as viable as cells without polypeptide over the timecourse of the experiment (5 h, see Supplementary Information). K₆₀L₂₀ vesicles were also found to be minimally cytotoxic. However, the K₆₀ homopolymer was found to be highly toxic to the HULEC-5A cells. For these samples, self assembly of the polycationic segments was found to greatly diminish their cytotoxicity, especially for the lysine polymers. These results are similar to those obtained for cationic hydrogel forming polypeptides, and appear to be a general phenomenon most likely resulting from chain assembly preventing free diffusion of the polycations to cell surfaces (Pakstis, L., Ozbas, B., Nowak, A.P., Deming, T.J., Pochan, D.J. The Effect of Chemistry and Morphology on the Biofunctionality of Self-Assembling Diblock Copolypeptide Hydrogels, Biomacromolecules 5, 312-318 (2004)). Thus, self-assembly of the R₆₀L₂₀ block copolymers into vesicles helps them function as effective intracellular delivery vehicles, despite the presence of large polycationic segments. This point was further demonstrated when T84 cells were incubated with R₆₀L₂₀ vesicles in serum containing media. The vesicles were found to transport into the cells despite the abundance of anionic serum proteins that typically bind and precipitate polycations such as polyarginine (Figure 3f) (Sela, M., Katchalski, E. Biological Properties of Poly α-Amino Acids, Adv. Protein Chem. 14, 391-478 (1959)). The construction of these vesicles from polypeptides provides a means to obtain synergy between structure and functionality within a single material, an approach that may prove widely applicable in the design and preparation of multifunctional materials.

### III. Conclusion

Vesicles composed of polyarginine and polyleucine segments that are stable in media, can entrap water soluble species, and can be processed to different sizes and prepared in large quantities have been prepared. The remarkable feature of these materials is that the polyarginine segments both direct structure for vesicle formation and provide functionality for efficient intracellular delivery of the vesicles. This unique synergy between nanoscale self-assembly and inherent peptide functionality provides a new approach for design of multifunctional materials for drug delivery.

## Claims

1. A vesicle comprising a shell encapsulating a polar fluid medium, wherein said shell comprises self-assembling block copolymers that include a first hydrophobic domain and a second hydrophilic intracellular transduction domain, wherein the hydrophilic domain contains a poly-arginine (polyR) domain.

2. The vesicle according to Claim 1, wherein said first hydrophobic domain ranges in length from about 10 to about 30 residues.

3. The vesicle according to Claim 1 or 2, wherein said first hydrophobic domain is a poly-leucine (polyL) domain.

4. The vesicle according to Claim 1, wherein said hydrophilic domain ranges in length from about 40 to about 80 residues.

5. The vesicle according to Claim 4, wherein said polyR domain is R₆₀.

6. The vesicle according to any one of the preceding claims, wherein said self-assembling block copolymer is a diblock copolypeptide, wherein said second domain has a length that is about 2 to 4 times longer than the length of said first domain.

7. The vesicle according to Claim 6, wherein said diblock copolypeptide is a R₆₀L₂₀.

8. The vesicle according to any one of the preceding claims, wherein said vesicle has a diameter ranging from about 50 to about 1000 nm.

9. The vesicle according to any one of the preceding claims, wherein said vesicle is stable at temperatures up to about 80°C.

10. The vesicle according to any one of the preceding claims, wherein said vesicle is non-toxic.

11. The vesicle according to any one of the preceding claims, wherein said polar fluid medium is an aqueous medium.

12. The vesicle according to Claim 11, wherein said aqueous medium comprises a water-soluble active agent.

13. A composition comprising a plurality of vesicles according to any one of the preceding claims.

14. A pharmaceutical composition comprising a vesicle according to any of claims 1 to 12, or comprising a plurality of vesicles according to claim 13, and at least one therapeutic or diagnostic agent, wherein the at least one therapeutic or diagnostic agent is encapsulated in the vesicle(s).

15. The composition according to claim 13 or claim 14 for use in therapy.

## Patentansprüche

1. Vesikel mit einer ein polares Fluidmedium einkapselnden Hülle, wobei die Hülle selbstassemblierende Blockcopolymere umfasst, die eine erste, hydrophobe Domäne und eine zweite, hydrophile intrazelluläre Transduktionsdomäne aufweisen, wobei die hydrophile Domäne eine Polyarginin-(polyR)-Domäne enthält.

2. Vesikel nach Anspruch 1, wobei die erste, hydrophobe Domäne in der Länge von ca. 10 bis ca. 30 Resten reicht.

3. Vesikel nach Anspruch 1 oder 2, wobei die erste, hydrophobe Domäne eine Polyleucin-(polyL)-Domäne ist.

4. Vesikel nach Anspruch 1, wobei die hydrophile Domäne in ihrer Länge von ca. 40 bis ca. 80 Resten reicht.

5. Vesikel nach Anspruch 4, wobei die polyR-Domäne R₆₀ ist.

6. Vesikel nach einem der vorhergehenden Ansprüche, das selbstassemblierende Blockcopolymer ein Diblock-Copolypeptid ist, wobei die zweite Domäne eine Länge hat, die etwa zwei- bis viermal länger als die Länge der ersten Domäne ist.

7. Vesikel nach Anspruch 6, wobei das Diblock-Copolypeptid ein R₆₀L₂₀ ist.

8. Vesikel nach einem der vorhergehenden Ansprüche, wobei das Vesikel einen Durchmesser von ca. 50 bis ca. 1000 nm hat.

9. Vesikel nach einem der vorhergehenden Ansprüche, wobei das Vesikel bei einer Temperatur bis ca. 80°C stabil ist.

10. Vesikel nach einem der vorhergehenden Ansprüche, wobei das Vesikel nichttoxisch ist.

11. Vesikel nach einem der vorhergehenden Ansprüche, wobei das polare Fluidmedium ein wässriges Medium ist.

12. Vesikel nach Anspruch 11, wobei das wässrige Medium einen wasserlöslichen Wirkstoff umfasst.

13. Zusammensetzung, umfassend eine Mehrzahl von Vesikeln gemäß einem der vorhergehenden Ansprüche.

14. Pharmazeutische Zusammensetzung, umfassend ein Vesikel gemäß einem der Ansprüche 1 bis 12 oder umfassend eine Mehrzahl von Vesikeln gemäß Anspruch 13 und mindestens ein therapeutisches oder diagnostisches Agens, wobei das mindestens eine therapeutische oder diagnostische Agens in dem(den) Vesikel(n) eingekapselt ist.

15. Zusammensetzung nach Anspruch 13 oder 14 zur therapeutischen Anwendung.

## Revendications

1. Vésicule comprenant une coquille dans laquelle est encapsulé un milieu fluide polaire, cette coquille comprenant des copolymères blocs à auto assemblage qui renferment un premier domaine hydrophobe et un second domaine de transduction intracellulaire hydrophile, le domaine hydrophile renfermant un domaine de poly-arginine (polyR).

2. Vésicule conforme à la revendication 1, dans laquelle le premier domaine hydrophobe à une longueur située dans la plage d'environ 10 à environ 30 résidus.

3. Vésicule conforme à la revendication 1 ou 2, dans laquelle le premier domaine hydrophobe est un domaine de poly-leucine (polyL).

4. Vésicule conforme à la revendication 1, dans laquelle le domaine hydrophile a une longueur située dans la plage comprise entre environ 40 et environ 80 résidus.

5. Vésicule conforme à la revendication 4, dans laquelle le domaine polyR est R₆₀.

6. Vésicule conforme à l'une quelconque des revendications précédentes, dans laquelle le copolymère bloc à auto assemblage est un copolypeptide di-blocs, le second domaine ayant une longueur qui est d'environ de 2 à 4 fois supérieure à la longueur du premier domaine.

7. Vésicule conforme à la revendication 6, dans laquelle le copolypeptide di-blocs est un R₆₀L₂₀.

8. Vésicule conforme à l'une quelconque des revendications précédentes, ayant un diamètre situé dans la plage comprise entre environ 50 à environ 1 000 nm.

9. Vésicule conforme à l'une quelconque des revendications précédentes, qui est stable à des températures allant jusqu'à environ 80°C.

10. Vésicule conforme à l'une quelconque des revendications précédentes, qui n'est pas toxique.

11. Vésicule conforme à l'une quelconque des revendications précédentes, dans laquelle le milieu fluide polaire est un milieu aqueux.

12. Vésicule conforme à la revendication 11, dans laquelle le milieu aqueux renferme un agent actif soluble dans l'eau.

13. Composition renfermant plusieurs vésicules conformes à l'une quelconque des revendications précédentes.

14. Composition pharmaceutique renfermant une vésicule conforme à l'une quelconque des revendications 1 à 12 ou renfermant plusieurs vésicules conformes à la revendication 13 et au moins un agent thérapeutique ou un agent de diagnostic, cet agent thérapeutique ou cet agent de diagnostic étant encapsulé dans la ou les vésicule(s).

15. Composition conforme à la revendication 13 ou à la revendication 14 destinée à être utilisée en thérapie.
